# EUROPEAN PATENT APPLICATION

(11) **EP 4 796 646 A1**
(43) Date of publication of application: **26.08.2026**
(21) Application number: 26158203.5
(22) Date of filing: 12.02.2026
(51) Int. Cl.: C12Q 1/6874

(54) **SOLUTION FOR SEQUENCING CHIP CLEANING, SEQUENCING METHOD, AND KIT**

(30) Priority: 19.02.2025 CN 202510185638
(71) Applicant: GeneMind Biosciences Company Limited, Shenzhen City, Guangdong 518023 (CN)
(72) Inventor: Shang, Huan, Shenzhen City, 518023 (CN); Han, Xuchen, Shenzhen City, 518023 (CN)
(74) Representative: Schiweck Weinzierl Koch Patentanwälte Partnerschaft mbB

(57) **Abstract**

The present application provides a solution for sequencing chip cleaning, a sequencing method, and a kit. The solution for sequencing chip cleaning includes a basic buffer, Na⁺ and/or K⁺, a surfactant, and a pH regulator. The solution further comprises at least one of a divalent cation, NH₄⁺, and a polymerase. In the solution for sequencing chip cleaning provided herein, at least one of a divalent cation, NH₄⁺, and a polymerase is further added on the basis of the basic buffer, Na⁺ and/or K⁺, the surfactant, and the pH regulator, such that, after a sequencing chip is cleaned by using the solution, the solution remaining on the sequencing chip can play a buffering role and provide a stable reaction environment for the incorporation of nucleotides or nucleotide analogs into nucleic acid templates when the sequencing chip is used for sequencing, thereby improving the reaction efficiency of incorporating nucleotides or nucleotide analogs into nucleic acid templates, further reducing the phasing, and improving the sequencing accuracy.

## Description

### TECHNICAL FIELD

The present application relates to the field of nucleic acid sequencing technology, and in particular, to a solution for sequencing chip cleaning, a sequencing method, and a kit.

### BACKGROUND

Next-generation sequencing, also referred to as high-throughput sequencing or massively parallel sequencing, enables the determination of nucleic acid sequences of multiple samples in one sequencing run. A common next-generation sequencing method is sequencing by synthesis (SBS). An SBS-based nucleic acid sequencing platform is based on the base pairing principle. It uses a DNA polymerase to attach a nucleotide or nucleotide analog to the 3' end of a sequencing primer bound to a nucleic acid template so as to controllably achieve single base extension, and acquires signal changes caused by the binding of each nucleotide or nucleotide analog, and further determines the base arrangement of the template on the basis of the changes in the acquired signals. Generally, the SBS method includes the following steps: (1) hybridizing a nucleic acid template of interest with a probe (or a sequencing primer) on the surface of a solid carrier (e.g., the substrate surface of a sequencing chip) to attach the nucleic acid template of interest to the surface of the solid carrier; (2) under the action of a DNA polymerase and in a condition suitable for the polymerase chain reaction, incorporating a nucleotide analog with a fluorophore and a cleavable blocking group into the nucleic acid template of interest by using the probe as the primer, so as to perform a single-base extension on the sequencing primer; (3) exciting the fluorophore on the nucleotide analog to emit light, and then imaging the surface to acquire luminescence signals on the surface; (4) removing the fluorophore and the cleavable blocking group in the nucleotide analog bound to the nucleic acid template of interest by using a cleavage reagent; and (5) repeating steps (2) to (4) described above to continue the extension of the sequencing primer, so as to form a complementary strand of the nucleic acid template of interest. Moreover, the SBS method may further include step (6): determining the type of nucleotide analogs incorporated into the nucleic acid template in each cycle of the extension by analyzing the optical signal obtained in step (3); and reading the introduced nucleotide analog types in sequence, and finally obtaining all nucleotide sequences of the nucleic acid template of interest. In step (2), the condition suitable for the polymerase chain reaction includes the presence of an extension reagent suitable for the polymerase chain reaction. It will be appreciated that the reaction system for incorporating the nucleotide analog into the nucleic acid template includes the extension reagent, thereby enabling the incorporation of the nucleotide analog into the nucleic acid template for the single-base extension on the sequencing primer.

During the process of incorporating the nucleotide analog into the nucleic acid template of interest, phase errors may easily occur due to the influence such as the reaction efficiency of incorporating the introduced nucleotide analog into the nucleic acid template of interest, etc. Generally, the phase error is represented by phasing (phase) or prephasing (prephase). The phasing refers to that the nucleotide analog that should have been incorporated into the nucleic acid template of interest in cycle N but participated in the reaction in cycle N+1. The prephasing refers to that the nucleotide analog that should have been incorporated into the nucleic acid template of interest in cycle N but participated in the reaction in cycle N-1. That is, there will be crosstalk between adjacent cycles in the same channel. Such phase errors may continuously accumulate and become stronger as the number of sequencing cycles increases. The final result is that four types of nucleotides are present simultaneously in one amplification cluster and are uniform in brightness. In this case, the base calling algorithm may not identify the correct sequencing signals in this cycle. That is, the sequence information of the nucleotide molecule of interest cannot be accurately acquired.

Therefore, reducing phasing or prephasing during the sequencing and improving sequencing accuracy are issues of concern.

### SUMMARY

The present application is intended to solve at least one of the existing technical problems in the prior art. Therefore, the present application provides a solution for sequencing chip cleaning, a sequencing method, and a kit, so as to improve the reaction efficiency of incorporating nucleotide or nucleotide analogs into a nucleic acid template, thereby reducing the phasing and improving the accuracy of sequencing.

In one aspect, the present application provides a solution for sequencing chip cleaning, including a basic buffer, Na⁺ and/or K⁺, a surfactant, and a pH regulator. The solution further includes at least one of a divalent cation, NH₄⁺, and a polymerase.

In the solution for sequencing chip cleaning provided herein, at least one of a divalent cation, NH₄⁺, and a polymerase is further added on the basis of the basic buffer, Na⁺ and/or K⁺, the surfactant, and the pH regulator, such that, after a sequencing chip is cleaned by using the solution, the solution remaining on the sequencing chip can play a buffering role and provide a stable reaction environment for the incorporation of nucleotides or nucleotide analogs into nucleic acid templates when the sequencing chip is used for sequencing, thereby improving the reaction efficiency of incorporating nucleotides or nucleotide analogs into nucleic acid templates, further reducing the phasing, and improving the sequencing accuracy.

In another aspect, the present application provides a sequencing method, including:
introducing a cleaning solution into a sequencing chip to clean the sequencing chip; and
introducing a first reaction solution into the cleaned sequencing chip to perform a first reaction, where
the sequencing chip includes a solid surface with a hybridization complex bound thereto, and the hybridization complex includes a nucleic acid template and a sequencing primer bound to the nucleic acid template;
the first reaction solution includes a polymerase and a nucleotide or nucleotide analog;
the first reaction includes incorporating the nucleotide or nucleotide analog into the nucleic acid template under the action of the polymerase;
the cleaning solution is the solution for sequencing chip cleaning described above; or the cleaning solution is a first reaction solution without the nucleotide or nucleotide analog.

By cleaning a sequencing chip with the cleaning solution, the solution remaining on the sequencing chip can play a buffering role and provide a stable reaction environment for the incorporation of nucleotides or nucleotide analogs into nucleic acid templates, thereby improving the reaction efficiency of incorporating nucleotides or nucleotide analogs into nucleic acid templates, further reducing the phasing, and improving the sequencing accuracy.

In yet another aspect, the present application provides a kit, including a cleaning solution, where the cleaning solution is the solution for sequencing chip cleaning according to the first aspect, or the cleaning solution is a first reaction solution without the nucleotide or nucleotide analog, and the first reaction solution includes at least one of a divalent cation, NH₄⁺, and a polymerase, and the nucleotide or nucleotide analog.

The additional aspects and advantages of the present application will be partially set forth in the following description, and will partially become apparent from the following description or be appreciated by practice of the present application.

### BRIEF DESCRIPTION OF THE DRAWINGS

FIG. 1 is a schematic view illustrating the sequencing results in a comparative example and an example according to the present application;
FIG. 2 is a schematic view illustrating the sequencing results in another example according to the present application.

### DETAILED DESCRIPTION

In order to make the technical problems, technical schemes, and beneficial effects to be solved by the present application more apparent, the present application will be further described in detail below with reference to the examples. It will be appreciated that the specific examples described herein are intended to illustrate the present application only, rather than limit the present application.

In the present application, the terms used in the examples of the present application are for the purpose of illustrating particular examples only and are not intended to limit the present application. The term "and/or" describes the associative relationship between associated objects, and refers to three possible relationships. For example, A and/or B may denote that: A is present alone, A and B are present simultaneously, and B is present alone. A and B may be singular or plural. The character "/" generally indicates an "and" relationship between the associated objects.

The term "at least one" means one or more, and "a plurality of" means two or more. The "at least one" or similar expressions thereof refer to any combination of these items, including any combination of the singular or plural items. For example, "at least one of a, b, or c" or "at least one of a, b, and c" may each refer to: a, b, c, a-b (i.e., a and b), a-c, b-c, or a-b-c, where each of a, b, and c may be a single item or a plurality of items.

As used in the examples and the appended claims of the present application, the singular forms "a", "an", and "the" are intended to include the plural forms as well, unless the context clearly indicates otherwise. The terms "first" and "second" are used for illustrative purposes only, are used for purposes distinguishing features such as substances, orientations, interfaces, messages, requests, and terminals from one another, and should not be construed as indicating or implying relative importance or implicitly indicating the number of the technical features indicated. Therefore, features defined by "first" and "second" may explicitly or implicitly include one or more of the features.

In the description of the present application, the concentration of the related components mentioned herein may not only refer to the specific content of each component, but also refer to the proportional relationship of the content among components. Therefore, as long as the content of the related components is scaled up or down according to the examples of the specification in the present application, it is within the scope disclosed in the examples of the specification in the present application.

The abbreviations used herein have their conventional meanings in the chemical and biological fields. The chemical structures and chemical formulas herein are constructed according to standard rules of chemical valence known in the chemical field.

In the embodiments of the present application, the term "sequencing chip" is also referred to as "chip", "biochip", "flow cell", "flow-cell", "flowcell", "reaction device", and the like, and such terms are interchangeable in expression. The sequencing chip is generally a sandwich structure of three layers (upper, middle, and lower layers), or a structure of two layers (upper and lower layers). The upper layer is a transparent glass layer, and the middle layer or the lower layer is a transparent or opaque substrate layer. At least one of the upper layer, the middle layer, or the lower layer is provided with one or more fluid channels arranged in an array. The fluid channels can receive liquids or solutions and provide physical space for reactions. The sequencing chip mentioned in the present application includes, but is not limited to, sequencing chips with the structure described above.

The term "cleaning" may be the removal of a liquid or solution, or the replacement with another liquid, solution, or other substance, which generally does not involve substantial processing and/or biochemical reactions.

The term "sequencing" is also referred to as "nucleic acid sequencing" or "gene sequencing" (i.e., the three expressions are interchangeable) and refers to the determination of the type and order of bases in a nucleic acid sequence, including sequencing by synthesis (SBS), sequencing by ligation (SBL), and/or the like, including DNA sequencing and/or RNA sequencing, including long fragment sequencing and/or short fragment sequencing (the long fragment and short fragment are defined relatively; for example, nucleic acid molecules longer than 1 kb, 2 kb, 5 kb, or 10 kb may be referred to as long fragments, and nucleic acid molecules shorter than 1 kb or 800 bp may be referred to as short fragments), and including double-end sequencing, single-end sequencing, paired-end sequencing, and/or the like (the double-end sequencing or paired-end sequencing may refer to the reading of any two segments or portions of the same nucleic acid template that are not completely overlapping); the sequencing involves the process of binding nucleotides or nucleotide analogs to the nucleic acid template and acquiring the corresponding signals emitted upon binding the nucleotides or nucleotide analogs to the nucleic acid template.

Sequencing generally involves multiple cycles of processes to achieve the determination of the order of multiple nucleotides/bases on the nucleic acid template, and each cycle of "a process to achieve the determination of the order of multiple nucleotides/bases on the nucleic acid template" may be referred to as "one cycle of sequencing" in the examples of the present application. One "cycle of sequencing", also referred to as "sequencing cycle", may be defined as the completion of the base extensions of four types of nucleotides/bases once, and in other words, one "cycle of sequencing" may be defined as the determination of the base type at any given position on the nucleic acid template. For sequencing platforms that achieve sequencing on the basis of polymerization or ligation reactions, one cycle of sequencing includes the process of binding four types of nucleotides (including nucleotide analogs) to the nucleic acid template at a time and acquiring the corresponding signals emitted; for platforms that achieve sequencing on the basis of polymerization reaction, the reaction system includes reaction substrate nucleotides or nucleotide analogs, a polymerase, and a nucleic acid template, a sequence fragment (a sequencing primer) is bound to the nucleic acid template, and on the basis of the principles of base pairing and polymerization reaction, the added reaction substrate nucleotides or nucleotide analogs are connected to the sequencing primer under the catalysis of the polymerase to achieve the binding of the nucleotides or nucleotide analogs to a specific position of the nucleic acid template, that is, the nucleotides or nucleotide analogs are incorporated into the nucleic acid template. Generally, one cycle of sequencing may include one or more base extensions (repeats). For example, four types of nucleotides are sequentially added to the reaction system to each perform base extension and corresponding acquisition of reaction signals, and one cycle of sequencing includes four base extensions; for another example, four types of nucleotides are added into the reaction system in any combinations (such as in pairs or in one-three combinations), the two combinations each perform base extension and corresponding acquisition of reaction signals, and one cycle of sequencing includes two base extensions; for yet another example, four types of nucleotides are added simultaneously to the reaction system for base extension and reaction signal acquisition, and one cycle of sequencing includes one base extension.

The term "nucleic acid template" may refer to a polymeric form of nucleotides of any length, and may include ribonucleotides, deoxyribonucleotides, analogs thereof, or mixtures thereof. The term may refer to a single-stranded or double-stranded polynucleotide. Nucleotides in a nucleic acid template may include natural nucleotides and functionally alternative analogs thereof. Examples of analogs can hybridize to nucleic acids in a sequence-specific manner, or can be used as templates for the replication of particular nucleotide sequences. Natural nucleotides generally have a backbone containing a phosphodiester bond. Analog structures may have alternative backbone linkages including any types known in the art. Natural nucleotides generally have deoxyribose (e.g., found in DNA) or ribose (e.g., found in RNA). Analog structures may have alternative sugar moieties including any types known in the art. Nucleotides may contain natural bases. Bases in natural DNA may include one or more of adenine, thymine, cytosine, and/or guanine, and bases in natural RNA may include one or more of adenine, uracil, cytosine, and/or guanine. Any non-natural base or base analog may also be contained in a nucleotide, such as a locked nucleic acid (LNA) and a bridged nucleic acid (BNA).

The term "sequencing primer" may be an oligonucleotide or a nucleic acid molecule that hybridizes to a target sequence of interest. In the embodiments, the sequencing primer serves as a substrate onto which nucleotides or nucleotide analogs may be polymerized by a polymerase. For example, the sequencing primer may be used as a starting point for DNA or RNA synthesis. For example, the sequencing primer may hybridize to a nucleic acid template to form a hybridization complex, so as to initiate the synthesis of a new strand complementary to the nucleic acid template. The sequencing primer may include any combination of nucleotides or analogs thereof. In some examples, the sequencing primer is a single-stranded oligonucleotide or a polynucleotide.

The term "blocking group" includes groups that can block a reaction site of a pentose in a nucleotide or nucleotide analog (e.g., 3'-OH) to prevent the polymerization of the nucleotide or nucleotide analog at the site, and the group can be removed by a chemical method or other methods to restore the reactivity of the reaction site.

The term "Q value", i.e., sequencing quality score, is used to evaluate the error detection rate of bases, wherein "Q30" represents that the error detection rate is 1/1000, corresponding to a detection accuracy of 99.9%. Reference is made to the following link for the definition of Q value:
https://www.illumina.com.cn/science/technology/next-generation-sequencing/plan-experimen ts/quality-scores.html.

As used herein, the "amplification" refers to the amplification of a nucleic acid template in a solid-phase or liquid-phase environment to give an amplification product of the nucleic acid template, e.g., an amplification cluster. The method for amplification is not limited. For example, the amplification may be achieved by PCR (thermocycling amplification) using the Taq enzyme or other enzymes, or by isothermal amplification techniques such as RPA (recombinase polymerase amplification), RCA (rolling circle amplification), SDA (strand displacement amplification) using Bst or Bsu or recombinase or a multi-enzyme system. For another example, a nucleic acid template can be amplified on a solid surface by bridge amplification (bridge PCR) or template walking amplification to form an amplicon on the surface; rolling circle amplification (RCA) in a liquid phase can also be used to acquire an amplification product of the nucleic acid template, and the amplification product is then loaded on the surface to form a DNA nanoball or the like on the surface.

By amplifying the nucleic acid template to form an amplification cluster, the signals generated when nucleotides or nucleotide analogs are incorporated into the nucleic acid template can be amplified during the sequencing, making it easier for a detection system, such as an optical imaging system, to detect the signal. However, due to the influence of factors such as the reaction efficiency of incorporating the introduced nucleotide or nucleotide analog into the nucleic acid template, phase errors such as phasing or prephasing may easily occur. That is, the sequencing reactions between different nucleic acid template molecules in the same amplification cluster gradually lose synchronization. Such phase errors continuously accumulate and become stronger as the number of sequencing cycles increases, which eventually causes serious interference to base calling, resulting in incorrect base calling, thereby affecting the sequencing read length and the sequencing accuracy.

One of the main reasons for the phasing phenomenon is the influence of the reaction efficiency of incorporating the introduced nucleotide or nucleotide analog into the nucleic acid template. Therefore, in the prior art, the reaction efficiency of incorporating the nucleotide or nucleotide analog into the nucleic acid template is usually improved by directly optimizing the formulation of the extension reagent, so as to achieve the purpose of reducing the phasing, or the formulation of the cleavage reagent is directly optimized to improve the efficiency of removing the fluorophore and the cleavable blocking group in the nucleotide analog bound to the nucleic acid template, thereby achieving the purpose of reducing the phasing and improving the gradual synchronization of the sequencing reactions between different nucleic acid template molecules in the same amplification cluster. However, due to the limitation of the association between the components in the extension reagent and the principle of the incorporation reaction, it is difficult to further reduce the phasing when the components in the extension reagent and the principle of the incorporation reaction remain unchanged; alternatively, due to the limitation of the association between the components in the cleavage reagent and the principle of the cleavage reaction, it is also difficult to further reduce the phasing when the components in the cleavage reagent and the principle of the cleavage reaction remain unchanged.

Based on the above knowledge, the inventor of the present application develops a new approach and transfers the development idea to the study of solutions for sequencing chip cleaning, i.e., the study of cleaning solutions. The sequencing chip provides physical space for accommodating liquids or solutions and sites for biochemical reactions, and the inventors expect that the purpose of reducing the phasing may be achieved by cleaning the sequencing chip with a cleaning solution before sequencing. After repeated studies on the cleaning solution, the inventor found that by optimizing the formulation of the cleaning solution and using the cleaning solution to clean the sequencing chip before sequencing, the cleaning solution remaining on the sequencing chip can play a buffering role after being mixed with the reaction system for incorporating the nucleotide or nucleotide analog into the nucleic acid template, which is beneficial to maintaining or increasing the concentration of the same components in the above reaction system as those in the cleaning solution and providing a stable reaction environment for the incorporation of the nucleotide or nucleotide analog into the nucleic acid template, thereby improving the reaction efficiency of incorporating the nucleotide or nucleotide analog into the nucleic acid template, reducing the phasing, and improving the sequencing accuracy. The reaction system for incorporating the nucleotide or nucleotide analog into the nucleic acid template includes an extension reagent and the like. In the following description, the extension reagent is also referred to as the first reaction solution. Illustratively, the extension reagent or the first reaction solution includes a divalent cation, NH₄⁺, a polymerase, a pH regulator, and a nucleotide or nucleotide analog. The nucleotide or nucleotide analog may be, for example, adenine A, cytosine C, guanine G, thymine T, uracil U, or a derivative thereof. The concentration of the divalent cation in the first reaction solution is 1-7 mmol/L. Illustratively, the concentration of the divalent cation in the first reaction solution may be 1 mmol/L, 1.5 mmol/L, 2 mmol/L, 2.5 mmol/L, 3 mmol/L, 3.5 mmol/L, 4 mmol/L, 4.5 mmol/L, 5 mmol/L, 5.5 mmol/L, 6 mmol/L, 6.5 mmol/L, 7 mmol/L, or a concentration between any two of the concentrations. The divalent cation may be, for example, selected from at least one of Mg²⁺ and Mn²⁺. More specifically, Mg²⁺ may be Mg²⁺ provided by a magnesium salt, and/or Mn²⁺ may be Mn²⁺ provided by a manganese salt. The concentration of NH₄⁺ in the first reaction solution is 20-150 mmol/L. Illustratively, the concentration of NH₄⁺ in the first reaction solution may be 20 mmol/L, 30 mmol/L, 40 mmol/L, 50 mmol/L, 60 mmol/L, 70 mmol/L, 80 mmol/L, 90 mmol/L, 100 mmol/L, 110 mmol/L, 120 mmol/L, 130 mmol/L, 140 mmol/L, 150 mmol/L, or a concentration between any two of the concentrations. NH₄⁺ may be NH₄⁺ provided by at least one of an ammonium salt and aqueous ammonia. The concentration of the polymerase in the first reaction solution is 0.02-0.1 ng/mL. Illustratively, the concentration of the polymerase in the first reaction solution is 0.02 ng/mL, 0.03 ng/mL, 0.04 ng/mL, 0.05 ng/mL, 0.06 ng/mL, 0.07 ng/mL, 0.08 ng/mL, 0.09 ng/mL, 0.1 ng/mL, or a concentration between any two of the concentrations. The polymerase may be selected from, for example, at least one of 9°N polymerase, Taq polymerase, Canace polymerase, Pfu polymerase, KOD polymerase, Phusion polymerase, Klenow polymerase, Bst polymerase, Phi29 polymerase, PrimerSTAR polymerase, and Tth polymerase. The pH regulator in the first reaction solution may be, for example, sodium hydroxide or potassium hydroxide, and is used for adjusting the pH of the first reaction solution to 8.8-9.3.

The embodiments of the present application provide a solution for sequencing chip cleaning, including a basic buffer, Na⁺ and/or K⁺, a surfactant, and a pH regulator. The solution further includes at least one of a divalent cation, NH₄⁺, and a polymerase.

In the solution for sequencing chip cleaning provided by the embodiments of the present application, at least one of a divalent cation, NH₄⁺, and a polymerase is further added on the basis of the basic buffer, Na⁺ and/or K⁺, the surfactant, and the pH regulator, such that, after a sequencing chip is cleaned by using the solution, the solution remaining on the sequencing chip can play a buffering role after being mixed with the reaction system for incorporating the nucleotide or nucleotide analog into the nucleic acid template, which is beneficial to maintaining or increasing the concentration of the same components in the above reaction system as those in the cleaning solution and providing a stable reaction environment for the incorporation of the nucleotide or nucleotide analog into the nucleic acid template. That is, in the embodiments of the present application, by utilizing the similarity in formulation between the cleaning solution and the first reaction solution of the above reaction system, the concentration of the components in the first reaction solution of the reaction system that are the same as those in the cleaning solution is maintained or increased, thereby improving the reaction efficiency of incorporating the nucleotide or nucleotide analog into the nucleic acid template, reducing the phasing, and improving the sequencing accuracy. In addition, the cleaning solution remaining on the sequencing chip can be used to adjust the above reaction system to a desired pH, thereby improving the reaction efficiency of incorporating the nucleotide or nucleotide analog into the nucleic acid template, reducing the phasing, and improving the sequencing accuracy.

In some embodiments, the basic buffer is selected from at least one of Tris buffer, Hepes buffer, glycine, ethanolamine, tetraethylethylenediamine, tetramethylethylenediamine, N-butyldiethanolamine, diethylaminoethanol, and N,N-dihydroxyethylglycine. The Tris buffer is the tris(hydroxymethyl)aminomethane buffer. The Hepes buffer is a nonionic amphoteric buffer, with its active ingredient being 2-[4-(2-hydroxyethyl)-1-piperazinyl]ethanesulfonic acid. The basic buffer can play a buffering role. When the pH of the above reaction system changes with the temperature change or the reaction progress, the basic buffer can maintain the relative stability of the pH of the above reaction system, such that the reaction of incorporating the nucleotide or nucleotide analog into the nucleic acid template can be performed at a desired pH, thereby improving the reaction efficiency, reducing the phasing, and improving the sequencing accuracy.

In some embodiments, the concentration of the basic buffer is 50-200 mmol/L. Illustratively, the concentration of the basic buffer may be 50 mmol/L, 55 mmol/L, 60 mmol/L, 65 mmol/L, 70 mmol/L, 75 mmol/L, 80 mmol/L, 85 mmol/L, 90 mmol/L, 95 mmol/L, 105 mmol/L, 110 mmol/L, 115 mmol/L, 120 mmol/L, 125 mmol/L, 130 mmol/L, 135 mmol/L, 140 mmol/L, 145 mmol/L, 150 mmol/L, 155 mmol/L, 160 mmol/L, 165 mmol/L, 170 mmol/L, 175 mmol/L, 180 mmol/L, 185 mmol/L, 190 mmol/L, 195 mmol/L, 200 mmol/L, or a concentration between any two of the concentrations.

In some embodiments, Na⁺ in the solution is Na⁺ provided by at least one of a sodium salt, sodium hydroxide, and an organic carboxylate salt of sodium. The sodium salt may be, for example, at least one of sodium halide and sodium sulfate; the organic carboxylate salt of sodium may be, for example, at least one of sodium formate and sodium acetate; the sodium halide may be, for example, at least one of sodium chloride, sodium iodide, and sodium bromide. Preferably, Na⁺ is Na⁺ provided by sodium hydroxide, and in addition to Na⁺, sodium hydroxide also provides OH⁻ to adjust the pH of the solution. The K⁺ in the solution is K⁺ provided by at least one of a potassium salt, potassium hydroxide, and an organic carboxylate salt of potassium. The potassium salt may be, for example, at least one of potassium halide and potassium sulfate; the potassium halide may be, for example, at least one of potassium chloride, potassium iodide, and potassium bromide; the organic carboxylate salt of potassium may be, for example, at least one of potassium acetate and potassium oxalate. Preferably, K⁺ is K⁺ provided by potassium hydroxide, and in addition to K⁺, potassium hydroxide also provides OH⁻ to adjust the pH of the solution.

In some embodiments, the concentration of Na⁺ and/or K⁺ is 10-100 mmol/L. Na⁺ and K⁺ can maintain the activity of the polymerase and the stability of the synthesized complementary strand of the nucleic acid template. Illustratively, the concentration of Na⁺ may be 10 mmol/L, 20 mmol/L, 30 mmol/L, 40 mmol/L, 50 mmol/L, 60 mmol/L, 70 mmol/L, 80 mmol/L, 90 mmol/L, 100 mmol/L, or a concentration between any two of the concentrations. Illustratively, the concentration of K⁺ may be 10 mmol/L, 20 mmol/L, 30 mmol/L, 40 mmol/L, 50 mmol/L, 60 mmol/L, 70 mmol/L, 80 mmol/L, 90 mmol/L, 100 mmol/L, or a concentration between any two of the concentrations.

In some embodiments, the surfactant in the solution can be used to eliminate bubbles in the solution and increase the stability of the solution system. The surfactant may be selected from, for example, at least one of Tween and Triton X-100.

In some embodiments, the volume percentage of the surfactant in the solution is 0.01%-0.1%. Illustratively, when the surfactant is Tween-20, its volume percentage may be 0.01%, 0.02%, 0.03%, 0.04%, 0.05%, 0.06%, 0.07%, 0.08%, 0.09%, 0.1%, or a volume percentage between any two of the volume percentages.

In some embodiments, the concentration of the divalent cation is 1-8 mmol/L. The configuration of the concentration of the divalent cation in the solution in the range of 1-8 mmol/L can make the concentration of the divalent cation in the solution close to or equal to the concentration of the divalent cation in the above reaction system, such that after the sequencing chip is cleaned with the solution, the solution remaining on the sequencing chip is prevented from diluting the above reaction system and reducing the concentration of the same divalent cation as that in the solution in the above reaction system and thus the reaction efficiency, thereby reducing the phasing and improving the sequencing accuracy. Illustratively, the concentration of the divalent cation may be 1 mmol/L, 1.5 mmol/L, 2 mmol/L, 2.5 mmol/L, 3 mmol/L, 3.5 mmol/L, 4 mmol/L, 4.5 mmol/L, 5 mmol/L, 5.5 mmol/L, 6 mmol/L, 6.5 mmol/L, 7 mmol/L, 7.5 mmol/L, 8 mmol/L, or a concentration between any two of the concentrations.

In some embodiments, the divalent cation is selected from at least one of Mg²⁺ and Mn²⁺. Divalent cations such as Mg²⁺ and Mn²⁺, as activity-assisting agents of the polymerase, help activate the activity of the polymerase, such that the polymerase catalyzes the formation of a phosphodiester bond between the 3'-OH of the sequencing primer bound to the nucleic acid template and the phosphate group of the nucleotide or nucleotide analog, thereby binding the nucleotide or nucleotide analog to the sequencing primer and binding the nucleotide or nucleotide analog to the nucleic acid template via complementary base pairing, i.e., incorporating the nucleotide or nucleotide analog into the nucleic acid template.

In some embodiments, Mg²⁺ is Mg²⁺ provided by a magnesium salt, and/or Mn²⁺ is Mn²⁺ provided by a manganese salt. That is, in the embodiments of the present application, the solution for sequencing chip cleaning includes the magnesium salt; and/or the solution for sequencing chip cleaning includes the manganese salt; alternatively, during the preparation of the solution for sequencing chip cleaning, the magnesium salt is added to provide Mg²⁺; and/or during the preparation of the solution for sequencing chip cleaning, the manganese salt is added to provide Mn²⁺.

In some embodiments, the concentration of NH₄⁺ is 50-200 mmol/L. Illustratively, the concentration of NH₄⁺ is 50 mmol/L, 60 mmol/L, 70 mmol/L, 80 mmol/L, 90 mmol/L, 100 mmol/L, 110 mmol/L, 120 mmol/L, 130 mmol/L, 140 mmol/L, 150 mmol/L, 160 mmol/L, 170 mmol/L, 180 mmol/L, 190 mmol/L, 200 mmol/L, or a concentration between any two of the concentrations. The configuration of the concentration of NH₄⁺ in the solution in the range of 50-200 mmol/L can make the concentration of NH₄⁺ in the solution close to or equal to the concentration of NH₄⁺ in the above reaction system, such that after the sequencing chip is cleaned with the solution, the solution remaining on the sequencing chip is prevented from diluting the above reaction system and reducing the concentration of NH₄⁺ in the above reaction system and thus the reaction efficiency, thereby reducing the phasing and improving the sequencing accuracy. Meanwhile, NH₄⁺ can also improve the reactivity of the polymerase, thereby improving the reaction efficiency of incorporating the nucleotide or nucleotide analog into the nucleic acid template, reducing the phasing, and improving the sequencing accuracy. The inventor speculates that the increase in the reactivity of the polymerase by NH₄⁺ may be associated with the ability of NH₄⁺ to change the charge distribution on the surface of the polymerase and/or to change the spatial configuration of the polymerase, such that the polymerase can be more easily bound to the reaction sites of the nucleic acid template and/or the sequencing primer and catalyzes the formation of a phosphodiester bond between the 3'-OH of the sequencing primer and the phosphate group of the nucleotide or nucleotide analog, thereby binding the nucleotide or nucleotide analog to the sequencing primer and binding the nucleotide or nucleotide analog to the nucleic acid template via complementary base pairing, i.e., incorporating the nucleotide or nucleotide analog into the nucleic acid template. In addition to increasing the reactivity of the polymerase, NH₄⁺ can weaken the non-specific binding of the nucleotide or nucleotide analog in the above reaction system to the hydrogen bond on the surface of the sequencing chip. The inventor speculates that this may be associated with the fact that NH₄⁺ can compete with the hydrogen bond on the surface of the sequencing chip for binding. NH₄⁺ competes with the hydrogen bond on the surface of the sequencing chip for binding, thereby weakening the non-specific binding of the nucleotide or nucleotide analog to the hydrogen bond on the surface of the sequencing chip. Moreover, NH₄⁺ can also play a buffering role. When the pH of the above reaction system changes with the temperature change or the reaction progress, NH₄⁺ can maintain the relative stability of the pH of the above reaction system, such that the reaction of incorporating the nucleotide or nucleotide analog into the nucleic acid template can be performed at a desired pH, thereby improving the reaction efficiency, reducing the phasing, and improving the sequencing accuracy.

In some embodiments, NH₄⁺ is NH₄⁺ provided by at least one of an ammonium salt and aqueous ammonia. That is, in the embodiments of the present application, the solution for sequencing chip cleaning includes at least one of the ammonium salt and aqueous ammonia; alternatively, during the preparation of the solution for sequencing chip cleaning, at least one of the ammonium salt and aqueous ammonia is added to provide NH₄⁺.

In some embodiments, the concentration of the polymerase is 0.08-0.1 ng/mL. Illustratively, the concentration of the polymerase is 0.08 ng/mL, 0.085 ng/mL, 0.09 ng/mL, 0.095 ng/mL, 0.1 ng/mL, or a concentration between any two of the concentrations. The configuration of the concentration of the polymerase in the solution in the range of 0.08-0.10 ng/mL can make the concentration of the polymerase in the solution close to or equal to the concentration of the polymerase in the above reaction system, such that after the sequencing chip is cleaned with the solution, the solution remaining on the sequencing chip is prevented from diluting the above reaction system and reducing the concentration of the polymerase in the above reaction system and thus the reaction efficiency. Meanwhile, during the process of sequencing chip cleaning using the solution, an immobilized enzyme may be formed, and the immobilization time of the enzyme is increased, thereby improving the reaction efficiency of incorporating the nucleotide or nucleotide analog into the nucleic acid template, reducing the phasing, and improving the sequencing accuracy.

In some embodiments, the polymerase is selected from at least one of 9°N polymerase, Taq polymerase, Canace polymerase, Pfu polymerase, KOD polymerase, Phusion polymerase, Klenow polymerase, Bst polymerase, Phi29 polymerase, PrimerSTAR polymerase, and Tth polymerase.

In some embodiments, the pH of the solution is between 8.8 and 9.7. Illustratively, the pH of the solution may be 8.8, 8.9, 9.0, 9.1, 9.2, 9.3, 9.4, 9.5, 9.6, 9.7, etc. The configuration of the pH of the solution in the range of 8.8-9.7 allows the adjustment of the pH of the above reaction system to a desired pH using the solution remaining on the sequencing chip when the pH of the above reaction system changes (the pH decreases) with the temperature change or the reaction progress, thereby improving the reaction efficiency of incorporating the nucleotide or nucleotide analog into the nucleic acid template, reducing the phasing, and improving the sequencing accuracy.

Since the pH of the reaction system will decrease as the temperature changes or as the reaction proceeds, in order to maintain or improve the reaction efficiency, it is a common practice to elevate the pH of the extension reagent or the first reaction solution in the reaction system in advance, for example, to 10. However, the inventors found that an increase in pH may cause a service life problem. For example, when the pH of the extension reagent or the first reaction solution reaches 10, the activity of the polymerase in the extension reagent or the first reaction solution may be significantly reduced, or the nucleotide or nucleotide analog may be damaged, which is not conducive to the storage of the extension reagent or the first reaction solution. A relatively lower pH may be more conducive to the storage and maintenance stability of the extension reagent or the first reaction solution. Therefore, in this embodiment, by adjusting the pH of the above reaction system to a desired pH by using the solution remaining on the sequencing chip, the service life problem due to the pH increase in the extension reagent or the first reaction solution in advance can be avoided, which is beneficial to the storage of the extension reagent or the first reaction solution.

In some embodiments, the pH of the solution may be adjusted to 8.8-9.7 using a pH regulator. The pH regulator may be selected from at least one of sodium hydroxide and potassium hydroxide. That is, in the embodiments of the present application, the solution for sequencing chip cleaning includes at least one of sodium hydroxide and potassium hydroxide; alternatively, during the preparation of the solution for sequencing chip cleaning, at least one of sodium hydroxide and potassium hydroxide is added.

In some embodiments, the solution further includes cystamine dihydrochloride. When the sequencing chip is washed with the solution including cystamine dihydrochloride, the remaining cystamine dihydrochloride on the sequencing chip helps remove the residual cleavage reagent in the above reaction system.

In some embodiments, the concentration of cystamine dihydrochloride is 20-200 mmol/L. Illustratively, the concentration of cystamine dihydrochloride may be 20 mmol/L, 30 mmol/L, 40 mmol/L, 50 mmol/L, 60 mmol/L, 70 mmol/L, 80 mmol/L, 90 mmol/L, 100 mmol/L, 110 mmol/L, 120 mmol/L, 130 mmol/L, 140 mmol/L, 150 mmol/L, 160 mmol/L, 170 mmol/L, 180 mmol/L, 190 mmol/L, 200 mmol/L, or a concentration between any two of the concentrations.

The use of the solution in the above embodiments of the present application to clean the sequencing chip before sequencing can significantly reduce the phasing phenomenon generated during sequencing and improve the sequencing accuracy.

Another embodiment of the present application provides a sequencing method, including:
introducing a cleaning solution into a sequencing chip to clean the sequencing chip; and
introducing a first reaction solution into the cleaned sequencing chip to perform a first reaction, where
the sequencing chip includes a solid surface with a hybridization complex bound thereto, and the hybridization complex includes a nucleic acid template and a sequencing primer bound to the nucleic acid template;
the first reaction solution includes a polymerase and a nucleotide or nucleotide analog;
the first reaction includes incorporating the nucleotide or nucleotide analog into the nucleic acid template under the action of the polymerase;
the cleaning solution is the solution for sequencing chip cleaning described above.

According to the sequencing method of the present application, by using the cleaning solution to clean the sequencing chip before sequencing, the cleaning solution remaining on the sequencing chip can play a buffering role after being mixed with the reaction system for incorporating the nucleotide or nucleotide analog into the nucleic acid template, which is beneficial to maintaining or increasing the concentration of the same components in the above reaction system as those in the cleaning solution and providing a stable reaction environment for the incorporation of the nucleotide or nucleotide analog into the nucleic acid template, thereby improving the reaction efficiency of incorporating the nucleotide or nucleotide analog into the nucleic acid template, reducing the phasing, and improving the sequencing accuracy.

In some embodiments, the nucleotide or nucleotide analog includes a detectable label, and the sequencing method further includes: after the first reaction is completed, introducing an imaging reagent into the sequencing chip to excite the detectable label, and acquiring a signal emitted by the detectable label. The "detectable label" includes, but is not limited to, optically detectable labels such as fluorophores CY3, CY5, CY7, ROX, Bodipy (BDP), Coumarin, and the like. The imaging reagent includes an antioxidant component, such as water-soluble vitamin E and the like. The imaging reagent can avoid or reduce the damage to or effect of light on the sample during the image acquisition process. After the first reaction is completed, the fluorophore is excited by laser to generate fluorescence, and the fluorescence signal is acquired by a detection system such as an optical imaging system to form an image. The type of the nucleotide analog incorporated into the nucleic acid template can be determined by analyzing the image, and the nucleotide sequence of the nucleic acid template can be acquired by sequentially reading the type of the incorporated nucleotide analog.

In some embodiments, after the first reaction is completed and before the imaging reagent is introduced into the sequencing chip, the cleaning solution is introduced into the sequencing chip to clean the sequencing chip, such that the cleaning solution replaces the first reaction solution of the previous step to provide a pre-buffering environment for the next cycle of sequencing.

In some embodiments, the nucleotide or nucleotide analog includes a cleavable blocking group, and the sequencing method further includes: after the acquisition of the signal emitted by the detectable label is completed, a cleavage reagent is introduced into the sequencing chip to remove the blocking group and the detectable label, so as to allow the incorporation of the next nucleotide or nucleotide analog into the nucleic acid template. As such, the nucleotide sequence of the nucleic acid template can be acquired by multiple cycles of extension reactions. After the blocking group and the detectable label are removed, the cleaning solution is introduced into the sequencing chip to clean the sequencing chip, such that the cleaning solution replaces the cleavage reagent and provides a pre-buffering environment for the next cycle of sequencing.

In some embodiments, before the first reaction solution is introduced into the cleaned sequencing chip to perform the first reaction, a second reaction solution is introduced into the sequencing chip to perform a second reaction, where the second reaction includes allowing the nucleic acid template in the sequencing chip to interact with the second reaction solution to achieve the amplification of the nucleic acid template. The second reaction solution includes components required by the amplification. By amplifying the nucleic acid template to form an amplification cluster, the fluorescence signals generated when nucleotides or nucleotide analogs are incorporated into the nucleic acid template can be amplified during the sequencing, making it easier for a detection system, such as an optical imaging system, to detect the fluorescence signal.

Yet another embodiment of the present application provides a sequencing method, including:
introducing a cleaning solution into a sequencing chip to clean the sequencing chip; and
introducing a first reaction solution into the cleaned sequencing chip to perform a first reaction, where
the sequencing chip includes a solid surface with a hybridization complex bound thereto, and the hybridization complex includes a nucleic acid template and a sequencing primer bound to the nucleic acid template;
the first reaction solution includes a polymerase and a nucleotide or nucleotide analog;
the first reaction includes incorporating the nucleotide or nucleotide analog into the nucleic acid template under the action of the polymerase;
the cleaning solution is a first reaction solution without the nucleotide or nucleotide analog.

By cleaning the sequencing chip with the first reaction solution without the nucleotide or nucleotide analog before sequencing, the sequencing method of the present application can provide a pre-buffering environment for the sequencing, which may facilitate maintaining or improving the concentration of components in the reaction system for incorporating the nucleotide or nucleotide analog into the nucleic acid template that are the same as those in the cleaning solution and ensure that the components in the first reaction solution including the nucleotide or nucleotide analog are not diluted by the cleaning solution and a high reaction concentration is kept to provide a stable reaction environment for the incorporation of the nucleotide or nucleotide analog into the nucleic acid template, thereby improving the reaction efficiency of incorporating the nucleotide or nucleotide analog into the nucleic acid template, reducing the phasing, and improving the sequencing accuracy.

In some embodiments, the nucleotide or nucleotide analog includes a detectable label, and the sequencing method further includes: after the first reaction is completed, introducing an imaging reagent into the sequencing chip to excite the detectable label, and acquiring a signal emitted by the detectable label. The "detectable label" includes, but is not limited to, optically detectable labels such as fluorophores CY3, CY5, CY7, ROX, Bodipy (BDP), Coumarin, and the like. The imaging reagent includes an antioxidant component, such as water-soluble vitamin E and the like. The imaging reagent can avoid or reduce the damage to or effect of light on the sample during the image acquisition process. After the first reaction is completed, the fluorophore is excited by laser to generate fluorescence, and the fluorescence signal is acquired by a detection system such as an optical imaging system to form an image. The type of the nucleotide analog incorporated into the nucleic acid template can be determined by analyzing the image, and the nucleotide sequence of the nucleic acid template can be acquired by sequentially reading the type of the incorporated nucleotide analog.

In some embodiments, after the first reaction is completed and before the imaging reagent is introduced into the sequencing chip, the cleaning solution is introduced into the sequencing chip to clean the sequencing chip, such that the cleaning solution replaces the first reaction solution of the previous step to provide a pre-buffering environment for the next cycle of sequencing.

In some embodiments, the nucleotide or nucleotide analog includes a cleavable blocking group, and the sequencing method further includes: after the acquisition of the signal emitted by the detectable label is completed, a cleavage reagent is introduced into the sequencing chip to remove the blocking group and the detectable label, so as to allow the incorporation of the next nucleotide or nucleotide analog into the nucleic acid template. As such, the nucleotide sequence of the nucleic acid template can be acquired by multiple cycles of extension reactions. After the blocking group and the detectable label are removed, the cleaning solution is introduced into the sequencing chip to clean the sequencing chip, such that the cleaning solution replaces the cleavage reagent and provides a pre-buffering environment for the next cycle of sequencing.

In some embodiments, before the first reaction solution is introduced into the cleaned sequencing chip to perform the first reaction, a second reaction solution is introduced into the sequencing chip to perform a second reaction, where the second reaction includes allowing the nucleic acid template in the sequencing chip to interact with the second reaction solution to achieve the amplification of the nucleic acid template. The second reaction solution includes components required by the amplification. By amplifying the nucleic acid template to form an amplification cluster, the fluorescence signals generated when nucleotides or nucleotide analogs are incorporated into the nucleic acid template can be amplified during the sequencing, making it easier for a detection system, such as an optical imaging system, to detect the fluorescence signal.

The embodiment of the present application provides a kit, including a cleaning solution, where the cleaning solution may be the solution for sequencing chip cleaning described in the above embodiments of the present application; or the cleaning solution is a first reaction solution without the nucleotide or nucleotide analog. The first reaction solution includes a divalent cation, NH₄⁺, and a polymerase, a pH regulator, and the nucleotide or nucleotide analog. The nucleotide or nucleotide analog may be, for example, adenine A, cytosine C, guanine G, thymine T, uracil U, or a derivative thereof.

In some embodiments, the concentration of the divalent cation in the first reaction solution is 1-7 mmol/L. Illustratively, the concentration of the divalent cation in the first reaction solution may be 1 mmol/L, 1.5 mmol/L, 2 mmol/L, 2.5 mmol/L, 3 mmol/L, 3.5 mmol/L, 4 mmol/L, 4.5 mmol/L, 5 mmol/L, 5.5 mmol/L, 6 mmol/L, 6.5 mmol/L, 7 mmol/L, or a concentration between any two of the concentrations.

In some embodiments, the divalent cation in the first reaction solution may be, for example, selected from at least one of Mg²⁺ and Mn²⁺. More specifically, Mg²⁺ may be Mg²⁺ provided by a magnesium salt, and/or Mn²⁺ may be Mn²⁺ provided by a manganese salt.

In some embodiments, the concentration of NH₄⁺ in the first reaction solution is 20-150 mmol/L. Illustratively, the concentration of NH₄⁺ in the first reaction solution may be 20 mmol/L, 30 mmol/L, 40 mmol/L, 50 mmol/L, 60 mmol/L, 70 mmol/L, 80 mmol/L, 90 mmol/L, 100 mmol/L, 110 mmol/L, 120 mmol/L, 130 mmol/L, 140 mmol/L, 150 mmol/L, or a concentration between any two of the concentrations.

In some embodiments, NH₄⁺ in the first reaction solution may be NH₄⁺ provided by at least one of an ammonium salt and aqueous ammonia.

In some embodiments, the concentration of the polymerase in the first reaction solution is 0.02-0.1 ng/mL. Illustratively, the concentration of the polymerase in the first reaction solution is 0.02 ng/mL, 0.03 ng/mL, 0.04 ng/mL, 0.05 ng/mL, 0.06 ng/mL, 0.07 ng/mL, 0.08 ng/mL, 0.09 ng/mL, 0.1 ng/mL, or a concentration between any two of the concentrations.

In some embodiments, the polymerase in the first reaction solution may be selected from, for example, at least one of 9°N polymerase, Taq polymerase, Canace polymerase, Pfu polymerase, KOD polymerase, Phusion polymerase, Klenow polymerase, Bst polymerase, Phi29 polymerase, PrimerSTAR polymerase, and Tth polymerase.

In some embodiments, the pH regulator in the first reaction solution may be, for example, sodium hydroxide or potassium hydroxide, and is used for adjusting the pH of the first reaction solution to 8.8-9.3.

By pumping the cleaning solution from the kit to clean the sequencing chip before or during the sequencing, the cleaning solution remaining on the sequencing chip can play a buffering role after being mixed with the reaction system for incorporating the nucleotide or nucleotide analog into the nucleic acid template, which is beneficial to maintaining or increasing the concentration of the same components in the above reaction system as those in the cleaning solution and providing a stable reaction environment for the incorporation of the nucleotide or nucleotide analog into the nucleic acid template, thereby improving the reaction efficiency of incorporating the nucleotide or nucleotide analog into the nucleic acid template, reducing the phasing, and improving the sequencing accuracy. In addition, the cleaning solution remaining on the sequencing chip can be used to adjust the above reaction system to a desired pH, such that the above reaction system reacts in a desired pH environment, thereby improving the reaction efficiency of incorporating the nucleotide or nucleotide analog into the nucleic acid template, reducing the phasing, and improving the sequencing accuracy. Moreover, cleaning the sequencing chip before sequencing using the first reaction solution without the nucleotide or nucleotide analog can provide a pre-buffering environment for the sequencing and ensure that components in the first reaction solution including the nucleotide or nucleotide analog are not diluted by the cleaning solution and a high reaction concentration is kept, thereby improving the reaction efficiency of incorporating the nucleotide or nucleotide analog into the nucleic acid template, reducing the phasing, and improving the sequencing accuracy.

The present application will be illustrated with reference to the following specific examples. The instruments and their manufacturers involved in the examples of the present application are shown in Table 1:

**Table 1**

| Instruments | Cat. No. | Manufacturer |
|---|---|---|
| FASTASeq300 sequencer | FASTASeq300 | Shenzhen GeneMind Biosciences Co., Ltd. |

### Example 1:

Cleaning solutions 1-1 and 1-2 were prepared according to the compositions shown in Table 2. The pH of cleaning solutions 1-1 and 1-2 was adjusted using sodium hydroxide.

**Table 2**

| Component | Cleaning solution 1-1 | Cleaning solution 1-2 |
|---|---|---|
| Tris buffer | 50 mM | 50 mM |
| Sodium chloride | 50 mM | 50 mM |
| Tween-20 | 0.01% | 0.01% |
| Cystamine dihydrochloride | 20 mM | 0 |
| pH | 7.0 - 7.4 | 9.2 - 9.6 |

### Control group

### Sequencing procedures:

The kit, the sequencing chip, and a library were prepared according to the requirements of the FASTAseq300 sequencer;
The library was immobilized on the sequencing chip for SE100 sequencing;
During the sequencing, with the reaction temperature in the sequencing chip being controlled at 60 °C, 75 µL of cleaning solution 1-2 and 122 µL of the first reaction solution were sequentially pumped into the sequencing chip from the kit (for 15 s of incubation). Then 50 µL of cleaning solution 1-2 and 122 µL of the imaging reagent were sequentially pumped into the sequencing chip, and the photographing was performed. After the photographing was completed, 30 µL of cleaning solution 1-2, 122 µL of the cleavage reagent (for 16 s of incubation), and 155 µL of cleaning solution 1-1 were sequentially pumped into the sequencing chip. The procedures were repeated until the sequencing was completed.

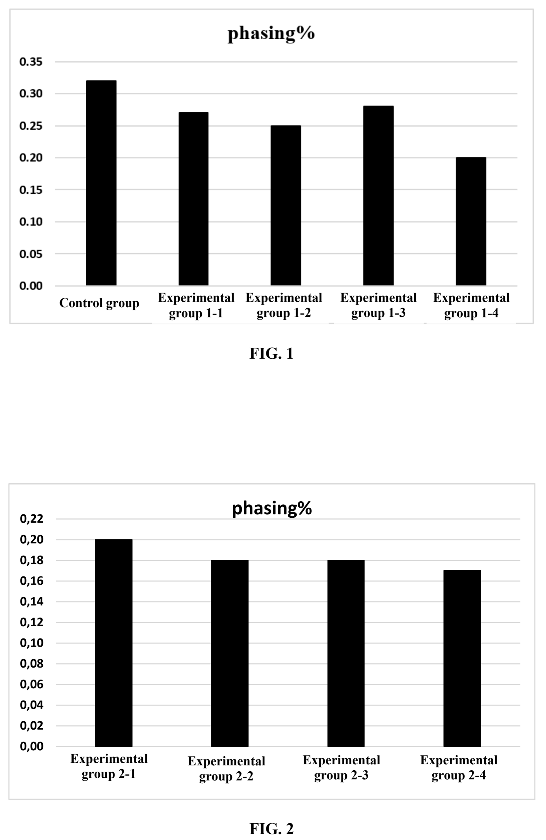

After the sequencing was completed, the sequencing results including Q30 value, phasing, prephasing, and mismatch ratio were summarized and analyzed, as shown in Table A and FIG. 1. The "mismatch ratio" refers to the proportion of unpaired bases in a nucleotide sequence to the total bases.

### Example 2

Cleaning solutions 2-1, 2-2, 2-3, and 2-4 were prepared according to the compositions shown in Table 3. The pH of cleaning solutions 2-1, 2-2, 2-3, and 2-4 was adjusted using sodium hydroxide.

**Table 3**

| Component | Cleaning solution 2-1 | Cleaning solution 2-2 | Cleaning solution 2-3 | Cleaning solution 2-4 |
|---|---|---|---|---|
| Tris buffer | 50 mM | 50 mM | 50 mM | 50 mM |
| Sodium chloride | 50 mM | 50 mM | 50 mM | 50 mM |
| Tween-20 | 0.01% | 0.01% | 0.01% | 0.01% |
| Magnesium sulfate | 0 mM | 0 mM | 5 mM | 5 mM |
| Ammonium sulfate | 0 mM | 120 mM | 0 mM | 120 mM |
| pH | 8.8 | 8.8 | 8.8 | 8.8 |

### Experimental group 1-1

### Sequencing procedures:

The kit, the sequencing chip, and a library were prepared according to the requirements of the FASTAseq300 sequencer;
The library was immobilized on the sequencing chip for SE100 sequencing;
During the sequencing, with the reaction temperature in the sequencing chip being controlled at 60 °C, 75 µL of cleaning solution 2-1 and 122 µL of the first reaction solution were sequentially pumped into the sequencing chip from the kit (for 15 s of incubation). Then 50 µL of cleaning solution 2-1 and 122 µL of the imaging reagent were pumped into the sequencing chip, and the photographing was performed. After the photographing was completed, 30 µL of cleaning solution 2-1, 122 µL of the cleavage reagent (for 16 s of incubation), and 155 µL of cleaning solution 1-1 were pumped into the sequencing chip. The procedures were repeated until the sequencing was completed.

After the sequencing was completed, the sequencing results including Q30 value, phasing, prephasing, and mismatch ratio were summarized and analyzed, as shown in Table A and FIG. 1.

### Experimental group 1-2

### Sequencing procedures:

The kit, the sequencing chip, and a library were prepared according to the requirements of the FASTAseq300 sequencer;
The library was immobilized on the sequencing chip for SE100 sequencing;
During the sequencing, with the reaction temperature in the sequencing chip being controlled at 60 °C, 75 µL of cleaning solution 2-2 and 122 µL of the first reaction solution were sequentially pumped into the sequencing chip from the kit (for 15 s of incubation). Then 50 µL of cleaning solution 2-2 and 122 µL of the imaging reagent were pumped into the sequencing chip, and the photographing was performed. After the photographing was completed, 30 µL of cleaning solution 2-2, 122 µL of the cleavage reagent (for 16 s of incubation), and 155 µL of cleaning solution 1-1 were pumped into the sequencing chip. The procedures were repeated until the sequencing was completed.

After the sequencing was completed, the sequencing results including Q30 value, phasing, prephasing, and mismatch ratio were summarized and analyzed, as shown in Table A and FIG. 1.

### Experimental group 1-3

### Sequencing procedures:

The kit, the sequencing chip, and a library were prepared according to the requirements of the FASTAseq300 sequencer;
The library was immobilized on the sequencing chip for SE100 sequencing;
During the sequencing, with the reaction temperature in the sequencing chip being controlled at 60 °C, 75 µL of cleaning solution 2-3 and 122 µL of the first reaction solution were sequentially pumped into the sequencing chip from the kit (for 15 s of incubation). Then 50 µL of cleaning solution 2-3 and 122 µL of the imaging reagent were pumped into the sequencing chip, and the photographing was performed. After the photographing was completed, 30 µL of cleaning solution 2-3, 122 µL of the cleavage reagent (for 16 s of incubation), and 155 µL of cleaning solution 1-1 were pumped into the sequencing chip. The procedures were repeated until the sequencing was completed.

After the sequencing was completed, the sequencing results including Q30 value, phasing, prephasing, and mismatch ratio were summarized and analyzed, as shown in Table A and FIG. 1.

### Experimental group 1-4

### Sequencing procedures:

The kit, the sequencing chip, and a library were prepared according to the requirements of the FASTAseq300 sequencer;
The library was immobilized on the sequencing chip for SE100 sequencing;
During the sequencing, with the reaction temperature in the sequencing chip being controlled at 60 °C, 75 µL of cleaning solution 2-4 and 122 µL of the first reaction solution were sequentially pumped into the sequencing chip from the kit (for 15 s of incubation). Then 50 µL of cleaning solution 2-4 and 122 µL of the imaging reagent were pumped into the sequencing chip, and the photographing was performed. After the photographing was completed, 30 µL of cleaning solution 2-4, 122 µL of the cleavage reagent (for 16 s of incubation), and 155 µL of cleaning solution 1-1 were pumped into the sequencing chip. The procedures were repeated until the sequencing was completed.

After the sequencing was completed, the sequencing results including Q30 value, phasing, prephasing, and mismatch ratio were summarized and analyzed, as shown in Table A and FIG. 1.

**Table A**

| | Density, K/mm² | Q30 | Phasing % | prephasing % | MismatchRatio % |
|---|---|---|---|---|---|
| Control group | 780.67 | 86.78 | 0.32 | 0.21 | 0.50 |
| Experimental group 1-1 | 789.00 | 89.30 | 0.27 | 0.22 | 0.30 |
| Experimental group 1-2 | 777.66 | 89.01 | 0.25 | 0.22 | 0.40 |
| Experimental group 1-3 | 792.19 | 89.54 | 0.28 | 0.23 | 0.43 |
| Experimental group 1-4 | 790.50 | 90.69 | 0.20 | 0.16 | 0.28 |

As can be seen from Table A and FIG. 1, when Mg²⁺ or NH₄⁺ is present in the cleaning solution, the phasing and base mismatch ratio can be reduced, and the Q30 value can be increased; when both Mg²⁺ and NH₄⁺ are present in the cleaning solution, the phasing and base mismatch ratio are reduced more significantly, and the Q30 value is also increased more significantly. This indicates that after the sequencing chip is cleaned with the cleaning solution, the cleaning solution remaining on the sequencing chip can play a buffering role after being mixed with the reaction system for incorporating the nucleotide or nucleotide analog into the nucleic acid template, which is beneficial to maintaining or increasing the concentration of the same components in the reaction system as those in the cleaning solution and providing a stable reaction environment for the incorporation of the nucleotide or nucleotide analog into the nucleic acid template, thereby improving the reaction efficiency of incorporating the nucleotide or nucleotide analog into the nucleic acid template, reducing the phasing, and improving the sequencing accuracy.

### Example 3

Cleaning solutions 3-1, 3-2, 3-3, and 3-4 were prepared according to the compositions shown in Table 4. The pH of cleaning solutions 3-1, 3-2, 3-3, and 3-4 was adjusted using sodium hydroxide.

**Table 4**

| Component | Cleaning solution 3-1 | Cleaning solution 3-2 | Cleaning solution 3-3 | Cleaning solution 3-4 |
|---|---|---|---|---|
| Tris buffer | 50 mM | 50 mM | 50 mM | 50 mM |
| Sodium chloride | 50 mM | 50 mM | 50 mM | 50 mM |
| Tween-20 | 0.01% | 0.01% | 0.01% | 0.01% |
| Magnesium sulfate | 2 mM | 2 mM | 2 mM | 2 mM |
| Ammonium sulfate | 80 mM | 80 mM | 80 mM | 80 mM |
| pH | 8.8 | 9.1 | 9.4 | 9.7 |

### Sequencing procedures:

### Experimental group 2-1:

The kit, the sequencing chip, and a library were prepared according to the requirements of the FASTAseq300 sequencer;
The library was immobilized on the sequencing chip for SE100 sequencing;
During the sequencing, with the reaction temperature in the sequencing chip being controlled at 60 °C, 75 µL of cleaning solution 3-1 and 122 µL of the first reaction solution were sequentially pumped into the sequencing chip from the kit (for 15 s of incubation). Then 50 µL of cleaning solution 3-1 and 122 µL of the imaging reagent were pumped into the sequencing chip, and the photographing was performed. After the photographing was completed, 30 µL of cleaning solution 3-1, 122 µL of the cleavage reagent (for 16 s of incubation), and 155 µL of cleaning solution 1-1 were pumped into the sequencing chip. The procedures were repeated until the sequencing was completed.

After the sequencing was completed, the sequencing results including Q30 value, phasing, prephasing, and mismatch ratio were summarized and analyzed, as shown in Table B and FIG. 2.

### Experimental group 2-2

The kit, the sequencing chip, and a library were prepared according to the requirements of the FASTAseq300 sequencer;
The library was immobilized on the sequencing chip for SE100 sequencing;
During the sequencing, with the reaction temperature in the sequencing chip being controlled at 60 °C, 75 µL of cleaning solution 3-2 and 122 µL of the first reaction solution were sequentially pumped into the sequencing chip from the kit (for 15 s of incubation). Then 50 µL of cleaning solution 3-2 and 122 µL of the imaging reagent were pumped into the sequencing chip, and the photographing was performed. After the photographing was completed, 30 µL of cleaning solution 3-2, 122 µL of the cleavage reagent (for 16 s of incubation), and 155 µL of cleaning solution 1-1 were pumped into the sequencing chip. The procedures were repeated until the sequencing was completed.

After the sequencing was completed, the sequencing results including Q30 value, phasing, prephasing, and mismatch ratio were summarized and analyzed, as shown in Table B and FIG. 2.

### Experimental group 2-3

The kit, the sequencing chip, and a library were prepared according to the requirements of the FASTAseq300 sequencer;
The library was immobilized on the sequencing chip for SE100 sequencing;
During the sequencing, with the reaction temperature in the sequencing chip being controlled at 60 °C, 75 µL of cleaning solution 3-3 and 122 µL of the first reaction solution were sequentially pumped into the sequencing chip from the kit (for 15 s of incubation). Then 50 µL of cleaning solution 3-3 and 122 µL of the imaging reagent were pumped into the sequencing chip, and the photographing was performed. After the photographing was completed, 30 µL of cleaning solution 3-3, 122 µL of the cleavage reagent (for 16 s of incubation), and 155 µL of cleaning solution 1-1 were pumped into the sequencing chip. The procedures were repeated until the sequencing was completed.

After the sequencing was completed, the sequencing results including Q30 value, phasing, prephasing, and mismatch ratio were summarized and analyzed, as shown in Table B and FIG. 2.

### Experimental group 2-4

The kit, the sequencing chip, and a library were prepared according to the requirements of the FASTAseq300 sequencer;
The library was immobilized on the sequencing chip for SE100 sequencing;
During the sequencing, with the reaction temperature in the sequencing chip being controlled at 60 °C, 75 µL of cleaning solution 3-4 and 122 µL of the first reaction solution were sequentially pumped into the sequencing chip from the kit (for 15 s of incubation). Then 50 µL of cleaning solution 3-4 and 122 µL of the imaging reagent were pumped into the sequencing chip, and the photographing was performed. After the photographing was completed, 30 µL of cleaning solution 3-4, 122 µL of the cleavage reagent (for 16 s of incubation), and 155 µL of cleaning solution 1-1 were pumped into the sequencing chip. The procedures were repeated until the sequencing was completed.

After the sequencing was completed, the sequencing results including Q30 value, phasing, prephasing, and mismatch ratio were summarized and analyzed, as shown in Table B and FIG. 2.

**Table B**

| | Density, K/mm² | Q30 | phasing % | prephasing % | MismatchRatio % |
|---|---|---|---|---|---|
| Experimental group 2-1 | 863.50 | 93.42 | 0.20 | 0.17 | 0.25 |
| Experimental group 2-2 | 908.00 | 93.69 | 0.18 | 0.15 | 0.29 |
| Experimental group 2-3 | 863.50 | 94.55 | 0.18 | 0.15 | 0.21 |
| Experimental group 2-4 | 865.00 | 93.04 | 0.17 | 0.16 | 0.30 |

As shown in Table B and FIG. 2, as the pH of the cleaning solution increased, the phasing gradually decreased, and the prephasing also gradually decreased. When the pH was 9.4, the phasing was reduced to 0.18%, the prephasing was reduced to 0.15%, and the base mismatch ratio was also significantly reduced. This indicates that after the sequencing chip is cleaned with the cleaning solution, the cleaning solution remaining on the sequencing chip can adjust the pH of the reaction system for incorporating the nucleotide or nucleotide analog into the nucleic acid template to provide the reaction system with an environment of the desired pH for performing the reaction, thereby improving the reaction efficiency of incorporating nucleotides or nucleotide analogs into nucleic acid templates, further reducing the phasing and prephasing, and improving the sequencing accuracy.

### Example 4

Cleaning solution 4 was prepared according to the compositions shown in Table 5. The pH of cleaning solution 4 was adjusted using sodium hydroxide.

**Table 5**

| Component | Cleaning solution 4 |
|---|---|
| Tris buffer | 50 mM |
| Sodium chloride | 50 mM |
| Tween-20 | 0.01% |
| Magnesium sulfate | 5 mM |
| Ammonium sulfate | 120 mM |
| Cystamine dihydrochloride | 50 mM |
| pH | 9.4 |

### Sequencing procedures:

### Experimental group 3

The kit, the sequencing chip, and a library were prepared according to the requirements of the FASTAseq300 sequencer;
The library was immobilized on the sequencing chip for SE100 sequencing;
During the sequencing, with the reaction temperature in the sequencing chip being controlled at 60 °C, 122 µL of the first reaction solution (for 15 s of incubation), 50 µL of cleaning solution 3-3, and 122 µL of the imaging reagent were sequentially pumped into the sequencing chip, and the photographing was performed. After the photographing was completed, 30 µL of cleaning solution 3-3, 122 µL of the cleavage reagent (for 16 s of incubation), and 155 µL of cleaning solution 4 were sequentially pumped into the sequencing chip. The procedures were repeated until the sequencing was completed.

After the sequencing was completed, the sequencing results including Q30 value, phasing, prephasing, and mismatch ratio were summarized and analyzed, as shown in Table C.

**Table C**

| | Density K/mm² | Q30 | phasing % | prephasing % | MismatchRatio % |
|---|---|---|---|---|---|
| Control group | 780.67 | 86.78 | 0.32 | 0.20 | 0.50 |
| Experimental group 2-3 | 863.50 | 94.55 | 0.18 | 0.15 | 0.21 |
| Experimental group 3 | 711.60 | 93.5 | 0.12 | 0.16 | 0.13 |

As can be seen from Table C, after the blocking group and the detectable label are cleaved from the cleavage reagent, cleaning the sequencing chip with the cleaning solution having a formula similar to that of the first reaction solution can significantly reduce the phasing and prephasing and increase the absolute value of Q30 by 6.7%. This indicates that when the cleaning solution is used to clean the sequencing chip, the reaction efficiency of incorporating the nucleotide or nucleotide analog into the nucleic acid template is improved, thereby reducing phasing and prephasing and improving the sequencing quality.

## Claims

1. A solution for sequencing chip cleaning, wherein
the solution comprises a basic buffer, Na⁺ and/or K⁺, a surfactant, and a pH regulator;
the solution further comprises at least one of a divalent cation, NH₄⁺, and a polymerase.

2. The solution for sequencing chip cleaning according to claim 1, wherein the concentration of the divalent cation is 1-8 mmol/L.

3. The solution for sequencing chip cleaning according to claim 1 or 2, wherein the divalent cation is selected from at least one of Mg²⁺ and Mn²⁺;
optionally, Mg²⁺ is Mg²⁺ provided by a magnesium salt; and/or,
Mn²⁺ is Mn²⁺ provided by a manganese salt.

4. The solution for sequencing chip cleaning according to any one of claims 1-3, wherein the concentration of NH₄⁺ is 50-200 mmol/L;
optionally, NH₄⁺ is NH₄⁺ provided by an ammonium salt or aqueous ammonia.

5. The solution for sequencing chip cleaning according to any one of claims 1-4, wherein the concentration of the polymerase is 0.08-0.1 ng/mL;
optionally, the polymerase is selected from at least one of 9°N polymerase, Taq polymerase, Canace polymerase, Pfu polymerase, KOD polymerase, Phusion polymerase, Klenow polymerase, Bst polymerase, Phi29 polymerase, PrimerSTAR polymerase, and Tth polymerase.

6. The solution for sequencing chip cleaning according to any one of claims 1-5, wherein the pH of the solution is 8.8-9.7;
optionally, the pH regulator is selected from at least one of sodium hydroxide and potassium hydroxide.

7. The solution for sequencing chip cleaning according to any one of claims 1-6, wherein the basic buffer is selected from at least one of Tris buffer, Hepes buffer, glycine, ethanolamine, tetraethylethylenediamine, tetramethylethylenediamine, N-butyldiethanolamine, diethylaminoethanol, and N,N-dihydroxyethylglycine.

8. The solution for sequencing chip cleaning according to any one of claims 1-7, wherein the surfactant is selected from at least one of Tween and Triton X-100.

9. The solution for sequencing chip cleaning according to any one of claims 1-8, further comprising cystamine dihydrochloride.

10. A sequencing method, comprising:
introducing a cleaning solution into a sequencing chip to clean the sequencing chip; and
introducing a first reaction solution into the cleaned sequencing chip to perform a first reaction, wherein
the sequencing chip comprises a solid surface with a hybridization complex bound thereto, and the hybridization complex comprises a nucleic acid template and a sequencing primer bound to the nucleic acid template;
the first reaction solution comprises a polymerase and a nucleotide or nucleotide analog;
the first reaction comprises incorporating the nucleotide or nucleotide analog into the nucleic acid template under the action of the polymerase;
the cleaning solution is the solution for sequencing chip cleaning according to any one of claims 1-9; or,
the cleaning solution is a first reaction solution without the nucleotide or nucleotide analog.

11. The sequencing method according to claim 10, wherein
the nucleotide or nucleotide analog comprises a detectable label;
the sequencing method further comprises:
after the first reaction is completed, introducing an imaging reagent into the sequencing chip to excite the detectable label, and acquiring a signal emitted by the detectable label;
optionally,, further comprising:
after the first reaction is completed and before the imaging reagent is introduced into the sequencing chip, introducing the cleaning solution into the sequencing chip to clean the sequencing chip.

12. The sequencing method according to claim 11, wherein
the nucleotide or nucleotide analog comprises a cleavable blocking group;
after the acquisition of the signal emitted by the detectable label is completed, a cleavage reagent is introduced into the sequencing chip to remove the blocking group and the detectable label;
optionally,
before the cleavage reagent is introduced into the sequencing chip, the cleaning solution is introduced into the sequencing chip to clean the sequencing chip.

13. The sequencing method according to claim 12, wherein
after the blocking group and the detectable label are removed, the cleaning solution is introduced into the sequencing chip to clean the sequencing chip;
optionally, before the first reaction solution is introduced into the cleaned sequencing chip to perform the first reaction, a second reaction solution is introduced into the sequencing chip to perform a second reaction, wherein the second reaction comprises allowing the nucleic acid template in the sequencing chip to interact with the second reaction solution to achieve the amplification of the nucleic acid template.

14. A kit, comprising a cleaning solution, wherein the cleaning solution is the solution for sequencing chip cleaning according to any one of claims 1-9; or the cleaning solution is a first reaction solution without the nucleotide or nucleotide analog, and the first reaction solution comprises a divalent cation, NH₄⁺, a polymerase, and the nucleotide or nucleotide analog.

15. The kit according to claim 14, wherein the concentration of the divalent cation is 1-7 mmol/L; optionally, the divalent cation is selected from at least one of Mg²⁺ and Mn²⁺;
optionally, Mg²⁺ is Mg²⁺ provided by a magnesium salt; and/or Mn²⁺ is Mn²⁺ provided by a manganese salt;
optionally, the concentration of NH₄⁺ is 20-150 mmol/L;
optionally, NH₄⁺ is NH₄⁺ provided by at least one of an ammonium salt and aqueous ammonia;
optionally, the concentration of the polymerase is 0.02-0.1 ng/mL;
optionally, the polymerase is selected from at least one of 9°N polymerase, Taq polymerase, Canace polymerase, Pfu polymerase, KOD polymerase, Phusion polymerase, Klenow polymerase, Bst polymerase, Phi29 polymerase, PrimerSTAR polymerase, and Tth polymerase;
optionally, the first reaction solution further comprises a pH regulator;
optionally, the pH regulator is selected from at least one of sodium hydroxide and potassium hydroxide;
optionally, the pH of the first reaction solution is 8.8-9.3.
